# EUROPÄISCHE PATENTANMELDUNG

(11) **EP 1 609 784 A1**
(43) Veröffentlichungstag der Anmeldung: **28.12.2005**
(21) Anmeldenummer: 04014917.1
(22) Anmeldetag: 25.06.2004
(51) Int. Cl.: C07D 271/07, C07D 401/12, A61K 31/4184, A61P 7/02

(54) **Verfahren zur Herstellung von 4-(Benzimidazolylmethylamino)-Benzamidinen**

(71) Anmelder: Boehringer Ingelheim Pharma GmbH & Co.KG, 55216 Ingelheim am Rhein (DE)
(72) Erfinder: Zerban, Georg Dr., 55218 Ingelheim (DE); Schmitt, Heinz-Peter, 55218 Ingelheim (DE)

(57) **Zusammenfassung**

Die Erfindung betrifft ein Verfahren zur Herstellung eines gegebenenfalls substituierten 4-Benzimidazol-2-ylmethylamino)-benzamidins, dadurch gekennzeichnet, dass man
(a) ein gegebenenfalls entsprechend substituiertes Diaminobenzol mit 2-[4-(1,2,4-Oxadiazol-5-on-3-yl)-phenylamino]-essigsäure kondensiert,
(b) das so erhaltene Produkt hydriert, und
(c) gegebenenfalls die Amidinogruppe carbonyliert.

## Beschreibung

### HINTERGRUND DER ERFINDUNG

### 1. TECHNISCHES GEBIET

Die Erfindung betrifft ein Verfahren zur Herstellung eines gegebenenfalls substituierten 4-(Benzimidazol-2-ylmethylamino)-benzamidins, wobei man
(a) ein gegebenenfalls entsprechend substituiertes Diaminobenzol mit 2-[4-(1,2,4-Oxadiazol-5-on-3-yl)-phenylamino]-essigsäure kondensiert,
(b) das so erhaltene Produkt hydriert, und
(c) gegebenenfalls die Amidinogruppe carbonyliert.

### 2. STAND DER TECHNIK

Substituierte (4-Benzimidazol-2-ylmethylamino)-benzamidine, insbesondere 1-Methyl-2-[*N*-[4-(*N*-n-hexyloxycarbonylamidino)phenyl]-amino-methyl]-benzimidazol-5-yl-carbonsäure-*N*-(2-pyridyl)-*N*-(2-ethoxycarbonylethyl)-amid sind bereits aus der Internationalen Patentanmeldung WO 98/37075 als Wirkstoffe mit einer Thrombin-hemmenden und die Thrombinzeit verlängernden Wirkung offenbart werden, bekannt.

Hauptindikationsgebiet der Verbindung der chemischen Formel I ist die postoperative Prophylaxe von tiefen Venenthrombosen.

In der WO 98/37075 wird vorgeschlagen die substituierten (4-Benzimidazol-2-ylmethylamino)-benzamidine durch Umsetzung der entsprechenden, substituierten (4-Benzimidazol-2-ylmethylamino)-benzonitrile mit Ammoniak herzustellen. Dieses Verfahren ist produktionstechnisch sehr aufwändig und führt zu einer hohen Belastung an zu entsorgenden Säuren.

Der vorliegenden Erfindung lag die Aufgabe zu Grunde eine Verfahrensvariante zur Herstellung der substituierten (4-Benzimidazol-2-ylmethylamino)-benzamidine aufzuzeigen, bei der diese produktionstechnisch aufwändige Verfahrensstufe vermieden werden kann.

### KURZE ZUSAMMENFASSUNG DER ERFINDUNG

Überraschenderweise wurde nun gefunden, dass die substituierten 4-(Benzimidazol-2-ylmethylamino)-benzamidine in hohen Ausbeuten und unter Einsatz kostengünstiger Hilfsstoffe hergestellt werden können, wenn man
(a) ein gegebenenfalls entsprechend substituiertes Diaminobenzol mit 2-[4-(1,2,4-Oxadiazol-5-on-3-yl)-phenylamino]-essigsäure kondensiert,
(b) das so erhaltene Produkt hydriert, und
(c) gegebenenfalls die Amidinogruppe, vorzugsweise mit einem Alkylhalogenformiat in Gegenwart einer Base, insbesondere mit Hexylchloroformiat carbonyliert.

Ein weiterer Gegenstand der Erfindung sind die bei dem erfindungsgemäßen Verfahren durchlaufenen, neuen Zwischenprodukte der Formel III: 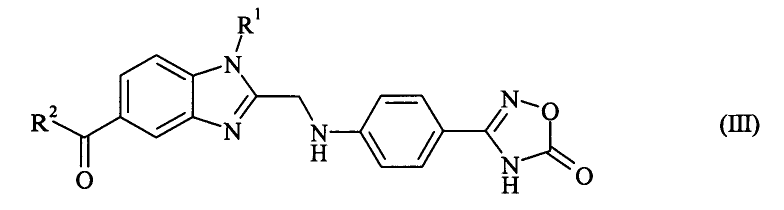 worin R¹ und R² für die nachfolgende Verbindungen der Formel (I) angegebene Bedeutung aufweisen, sowie
2-[4-(1,2,4-Oxadiazol-5-on-3-yl)-phenylamino]-essigsäure, und
2-[4-(1,2,4-Oxadiazol-5-on-3-yl)-anilin.

### DETAILLIERTE BESCHREIBUNG DER ERFINDUNG

Bevorzugt ist Verfahren zur Herstellung eines gegebenenfalls substituierten 4-(Benzimidazol-2-ylmethylamino)-benzamidins der Formel (I) 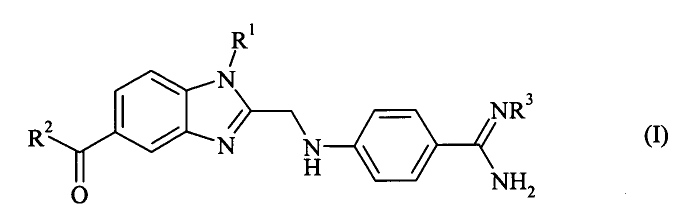 worin
R¹ für C₁₋₆-Alkyl- oder C₃₋₇-Cycloalkylgruppe steht,
R²
(i) für eine C₁₋₆-Alkylgruppe, eine gegebenenfalls durch eine C₁₋₃-Alkylgruppe substituierte C₃₋₇-Cycloalkylgruppe, wobei die C₁₋₃-Alkylgruppe zusätzlich durch eine Carboxylgruppe oder durch eine *in-vivo* in eine Carboxygruppe überführbare Gruppe substituiert sein kann,
   steht, oder
(ii) für eine R²¹NR²²-Gruppe steht, in der
   R²¹ eine C₁₋₆Alkylgruppe, die durch eine Carboxy-, C₁₋₆ Alkoxycarbonyl-, Benzyloxycarbonyl-, C₁₋₃-Alkylsulfonylaminocarbonyl-, Phenylsulfonylaminocarbonyl-, Trifluorsulfonylamino-, Trifluorsulfonylaminocarbonyl- oder 1H-Tetrazolylgruppe substituiert sein kann,
      eine durch eine Hydroxy-, Phenyl-C₁₋₃-alkoxy-, Carboxy-C₁₋₃-alkylamino-, C₁₋₃-Alkoxycarbonyl-C₁₋₃-alkylamino-, N-(C₁₋₃-Alkyl)-carboxy-C₁₋₃-alkylamino- oder N-(C₁₋₃-Alkyl)-C₁₋₃-alkoxycarbonyl-C₁₋₃-alkylaminogruppe substituierte C₂₋₄-Alkylgruppe, wobei in den vorstehend erwähnten Gruppen das zum benachbarten Stickstoffatom stehende α-Kohlenstoffatom nicht substituiert sein kann, oder eine gegebenenfalls durch eine C₁₋₃-Alkylgruppe substituierte Piperidinylgruppe bedeutet, und
   R²² ein Wasserstoffatom, eine C₁₋₆-Alkylgruppe, eine gegebenenfalls durch eine C₁₋₃-Alkylgruppe substituierte C₃₋₇-Cycloalkylgruppe, eine C3₋₆-Alkenyl- oder C₃₋₆-Alkinylgruppe, wobei der ungesättigte Teil nicht direkt mit dem Stickstoffatom der R²¹NR²²-Gruppe verknüpft sein kann, eine gegebenenfalls durch ein Fluor-, Chlor- oder Bromatom, durch eine C₁₋₃-Alkyl- oder C₁₋₃-Alkoxygruppe substituierte Phenylgruppe, eine gegebenenfalls durch eine C₁₋₃-Alkylgruppe substituierte Benzyl-, Oxazolyl-, Isoxazolyl-, Thiazolyl-, Isothiazolyl-, Pyrazolyl-, Pyridinyl-, Pyrimidinyl-, Pyrazinyl-, Pyridazinyl-, Pyrrolyl-, Thienyl- oder oder Imidazolylgruppe bedeutet,oder
   R²¹ und R²² zusammen mit dem dazwischenliegenden Stickstoffatom eine gegebenenfalls durch eine Carboxy- oder C₁₋₄-Alkoxycarbonylgruppe substituierte 5- bis 7-gliedrige Cycloalkyleniminogruppe, an welche zusätzlich ein Phenylring ankondensiert sein kann, darstellen,
      und
R³ für ein Wasserstoffatom, eine C₁₋₉-Alkoxycarbonyl-, Cyclohexyloxycarbonyl-, Phenyl-C₁₋₃-alkoxycarbonyl-, Benzoyl-, p-C₁₋₃Alkyl-benzoyl- oder Pyridinoylgruppe steht, wobei der Ethoxyteil in 2-Stellung der vorstehend erwähnten C₁₋₉-Alkoxycarbonylgruppe zusätzlich durch eine C₁₋₃-Alkylsulfonyl- oder 2-(C₁₋₃-Alkoxy)-ethylgruppe substituiert sein kann, darstellt,
   wobei man im Schritt (a) ein Phenyldiamin der Formel (II) 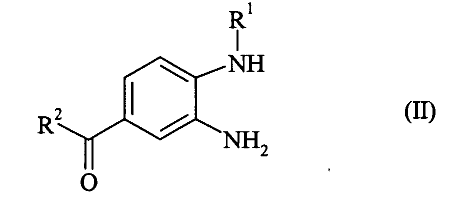 worin R¹ und R² die für Formel (I) angegebene Bedeutung aufweisen,
   mit 2-[4-(1,2,4-Oxadiazol-5-on-3-yl)-phenylamino]-essigsäure umsetzt, das erhaltene Produkt der Formel (III) 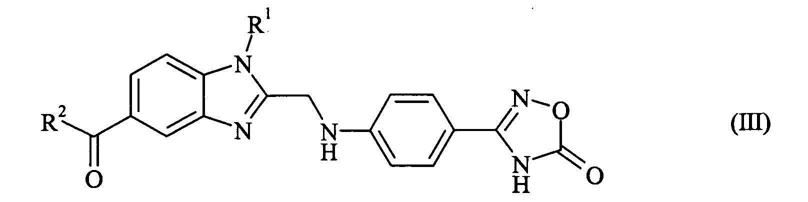 worin R¹ und R² die für Formel (I) angegebene Bedeutung aufweisen,
   in Schritt (b) hydriert, und
   (c) gegebenenfalls die so erhaltene Verbindung der Formel (I), worin R³ Wasserstoff bedeutet, mit einer Verbindung der Formel (IV)

      R³-X (IV)

      worin R³ die für Formel (I) angegebene Bedeutung aufweist, und
      X für eine geeignete Abgangsgruppe steht,
      umsetzt.

Besonders bevorzugt ist das erfindungsgemäße Verfahren zur Herstellung der Verbindungen der Formel (I), worin,
R¹ für eine C₁₋₃-Alkylgruppe steht,
R² für eine R²¹NR²²-Gruppe steht, in der
R²¹ eine C₁₋₃ Alkylgruppe, die durch eine Carboxy-, C₁₋₃ Alkoxycarbonyl-, substituiert sein kann,
bedeutet, und
R²² ein Wasserstoffatom, eine C₁₋₃-Alkylgruppe, eine gegebenenfalls durch eine C₁₋₃-Alkylgruppe substituierte Pyridinylgruppe bedeutet,
und
R³ für ein Wasserstoffatom, eine C₁₋₈-Alkoxycarbonylgruppe steht.

Am meisten bevorzugt ist das erfindungsgemäße Verfahren zur Herstellung der Verbindung der Formel (I), worin,
R¹ für eine Methylgruppe steht,
R² für eine R²¹NR²²-Gruppe steht, in der
R²¹ eine Ethylgruppe, die durch eine Ethoxycarbonylgruppe substituiert ist, bedeutet, und
R²² Pyridin-2-ylgruppe bedeutet,
und
R³ für eine Hexyloxycarbonylgruppe steht.

Die folgenden Ausführungsformen (A) bis (E) des erfindungsgemäßen Verfahrens sind bevorzugt:
(A) die Kondensation des Schrittes (a) wird in Gegenwart eines inerten Verdünnungsmittels und eines wasserbindenden Mittels durchgeführt.

Die entsprechend substituiertes Diaminobenzole der Formel (II) sind z.B. aus der Internationalen Patentanmeldung WO 98/37075 bekannt oder können in Analogie zu den dort beschriebenen hergestellt werden. Besonders bevorzugt werden 3-Amino-4-methylaminobenzoesäure-amide, insbesondere 3-Amino-4-methylaminobenzoesäure-*N*-(2-pyridyl)-*N*-(2-ethoxycarbonylethyl)-amid eingesetzt.

Als inerte Verdünnungsmittel können sowohl aprotische apolare Lösungsmittel - wie z.B. aliphatische oder aromatische, gegebenenfalls halogenierte Kohlenwasserstoffe - oder aprotische polare Lösungsmittel wie z.B. Ether und/oder Amide bzw. Lactame und/oder Gemische davon eingesetzt werden. Als aprotische apolare Lösungsmittel werden bevorzugt verzweigte oder unverzweigte C₅ - C₈ aliphatische Alkane, C₄ - C₁₀ Cycloalkane, C₁ - C₆ aliphatische Halogenalkane, C₆ - C₁₀ aromatische Alkane oder Gemische davon eingesetzt. Besonders bevorzugt werden Alkane wie Pentan, Hexan oder Heptan, Cycloalkane wie Cyclohexan oder Methylcyclohexan, Halogenalkane wie Dichlormethan, aromatische Alkane wie Benzol, Toluol oder Xylol oder deren Mischungen eingesetzt. Als aprotische Lösungsmittel eignen sich polare Ether wie beispielsweise Tetrahydrofuran (THF), *tert*-Butyl-Methylether oder Dimethoxyethylether oder Amide wie beispielsweise Dimethylformamid, oder Lactame wie beispielsweise N-Methylpyrrolidon.

Als wasserbindende Mittel können hygroskopische Salze, anorganische oder organische Säuren, Anhydride von anorganischen oder organischen Säuren, Molsiebe oder Harnstoffderivate eingesetzt werden. Besonders bevorzugt ist 1,1 '-Carbonyldiimidazol.

In einer besonders bevorzugten Ausführungsform wird 1,1'-Carbonyldiimidazol in THF suspendiert und erwärmt. 2-[4-(1,2,4-Oxadiazol-5-on-3-yl)-phenylamino]-essigsäure wird zugegeben. Das entsprechend substituierte Diaminobenzol wird in THF hinzugefügt.
Die Reaktionsmischung wird bei etwa 50°C gerührt und anschließend nach Zugabe von Essigester eingeengt.
(B) die Hydrierung des Schrittes (b) wird in Gegenwart eines inerten Verdünnungsmittels und eines Hydrierkatalysators durchgeführt.

Besonders bevorzugt ist ein Verfahren, wobei die Hydrierung in einem Temperaturbereich von 0°C bis 100°C, vorzugsweise von 0°C bis 50°C, insbesondere von 10°C bis 30°C durchgeführt wird.

Weiterhin bevorzugt ist ein Verfahren, wobei die Hydrierung unter einem Druck von mehr als 0,5 bar bis 100 bar, vorzugsweise unter einem Druck von 1 bar bis 10 bar, insbesondere bei etwa 1 - 2 bar durchgeführt wird.

Als inerte Verdünnungsmittel können sowohl protische Lösungsmittel - wie z.B. Alkohole, Carbonsäuren und/oder Wasser - oder aprotische polare Lösungsmittel wie z.B. Ether und/oder Amide bzw. Lactame und/oder Gemische davon eingesetzt werden. Allen Lösungsmitteln kann gegebenenfalls Wasser zugesetzt sein. Als protische Lösungsmittel werden bevorzugt verzweigte oder unverzweigte C₁ - C₈ Alkanole, C₁- C₃ Carbonsäuren oder Gemische davon eingesetzt. Besonders bevorzugt werden niedere Alkohole wie Methanol, Ethanol, n-Propanol und Isopropanol, Carbonsäuren wie Ameisensäure, Essigsäure und Propionsäure oder deren Mischungen eingesetzt. Besonders bevorzugt wird als Reaktionsmedium Ethanol und/oder Essigsäure verwendet, wobei diese gegebenenfalls Wasser enthalten können. Als aprotische Lösungsmittel eignen sich polare Ether wie beispielsweise Tetrahydrofuran oder Dimethoxyethylether oder Amide wie beispielsweise Dimethylformamid, oder Lactame wie beispielsweise N-Methylpyrrolidon. Bevorzugt werden Lösungsmittel eingesetzt, die wenig zur Brennbarkeit neigen.

Als Hydrierkatalysatoren sind in der Regel Übergangsmetalle wie zum Beispiel Nickel, Platin oder Palladium oder deren Salze oder Oxide geeignet. Bevorzugt sind Raney Nickel, Platinoxid und Palladium auf einem inerten Trägermaterial, insbesondere Palladium auf Aktivkohle (Pd/C).

Bevorzugt sind solche Verfahren, wobei man das Produkt des Schrittes (a) zum Hydrierkatalysator bei der Hydrierung in einem Gew.-Verhältnis von 1:1 bis 1000:1, vorzugsweise von 5:1 bis 100:1 einsetzt.

In einer besonders bevorzugten Ausführungsform wird das Produkt des Schritts (a)in Ethanol aufgenommen und nach Zugabe von Essigsäure und bei 2 bar Wasserstoff mit wasserfeuchtem 10%-igem Pd/C bei Raumtemperatur hydriert. Man filtriert vom Katalysator und gibt p-Toluolsulfonsäure, gelöst in 90 ml Ethanol zu. Das Tosylat des erhaltenen 4-(Benzimidazol-2-ylmethylamino)-benzamidins fällt aus, wird abfiltriert und in mehreren Portionen mit Ethanol nachgewaschen.
(C) Zur Herstellung von 2-[4-(1,2,4-Oxadiazol-5-on-3-yl)-phenylamino]-essigsäure wird 2-[4-(1,2,4-Oxadiazol-5-on-3-yl)-anilin mit einem 2-Halogenessigsäureester, vorzugsweise Bromessigsäureethylester, in Gegenwart einer schwachen Base, vorzugsweise ein tertiäres Amin, wie zum Beispiel Triethylamin oder ein Alkalimetallcarbonat, wie zum Beispiel Natriumcarbonat in einem inerten Lösungsmittel umgesetzt, und der erhaltene 2-[4-(1,2,4-Oxadiazol-5-on-3-yl)-phenylamino]-essigsäureester verseift.

Als inerte Verdünnungsmittel können sowohl protische Lösungsmittel - wie z.B. Alkohole, und/oder Wasser - oder aprotische polare Lösungsmittel wie z.B. Ether und/oder Amide bzw. Lactame und/oder Gemische davon eingesetzt werden. Allen Lösungsmitteln kann gegebenenfalls Wasser zugesetzt sein. Als protische Lösungsmittel werden bevorzugt verzweigte oder unverzweigte C₁ - C₈ Alkanole oder Gemische davon eingesetzt. Besonders bevorzugt werden niedere Alkohole wie Methanol, Ethanol, n-Propanol und Isopropanol oder deren Mischungen eingesetzt. Besonders bevorzugt wird als Reaktionsmedium Isopropanol verwendet, wobei dieses gegebenenfalls Wasser enthalten kann. Als aprotische Lösungsmittel eignen sich polare Ether wie beispielsweise Tetrahydrofuran oder Dimethoxyethylether oder Amide wie beispielsweise Dimethylformamid, oder Lactame wie beispielsweise N-Methylpyrrolidon.

In einer besonders bevorzugten Ausführungsform wird Bromessigsäureethylester zu einer Suspension von 2-[4-(1,2,4-Oxadiazol-5-on-3-yl)-anilin und Natriumcarbonat in Wasser/Isopropanol zudosiert und gerührt. Die gekühlte Suspension wird abgesaugt, in mehreren Portionen mit Wasser und Ethanol nach gewaschen und getrocknet.

Die Verseifung erfolgt vorzugsweise in einem protischen Lösungsmittel mit einem Alkali oder Erdalkalihydroxid, insbesondere mit Lithium-, Natrium oder Kaliumhydroxid.

In einer besonders bevorzugten Ausführungsform wird 2-[4-(1,2,4-Oxadiazol-5-on-3-yl)-phenylamino]-essigsäureester in Wasser suspendiert und bei Raumtemperatur langsam mit einer wässrigen Lösung von NaOH versetzt. Die Suspension geht in eine Lösung über und wird auf 45 bis 75°C erwärmt. Die so erhaltene Lösung wird mit HCl versetzt, bis etwa pH 5 erreicht ist. Der Feststoff wird isoliert und mit kaltem Wasser sowie kaltem Ethanol und MtBE gewaschen.
(D) zur Herstellung von 2-[4-(1,2,4-Oxadiazol-5-on-3-yl)-anilin wird 4-Aminophenyl-amidoxim mit einem Dialkylcarbonat, vorzugsweise Dimethylcarbonat oder Diethylcarbonat in Gegenwart einer Base, vorzugsweise eines Alkalimetallalkoholats, insbesondere Natriummethylat, Natriumethylat oder Kalium *tert*-Butanolat umgesetzt.

4-Aminophenyl-amidoxim kann z.B. durch Umsetzung von 4-Aminobenzonitril mit Hydroxylamin Hydrochlorid hergestellt werden.

In einer besonders bevorzugten Ausführungsform wird Natriummethylat bei 70-75°C zu einer Suspension von 4-Aminophenyl-amidoxime in Ethanol gegeben und mit Ethanol nachgespült. Nach 15 min Rühren wird Diethylcarbonat zugetropft. Nach 2-4 Stunden Reaktionszeit wird abgekühlt und Ethanol bei 120 mbar und 40°C abdestilliert. Der Rückstand wird in Wasser aufgenommen nach Erwärmen durch halb konz. Natronlauge auf pH 10-12, dann durch Ansäuern mit konz. Salzsäure auf pH 5-6 eingestellt und langsam abgekühlt. Die Lösung geht in eine Suspension über, die filtriert und mehrmals mit kaltem Wasser und Ethanol nach gewaschen wird.

Die Herstellung der als Zwischenprodukt benötigten 2-[4-(1,2,4-Oxadiazol-5-on-3-yl)-phenylamino]-essigsäure aus 4-Aminobenzonitril wird im nachfolgenden Reaktionsschema dargestellt:

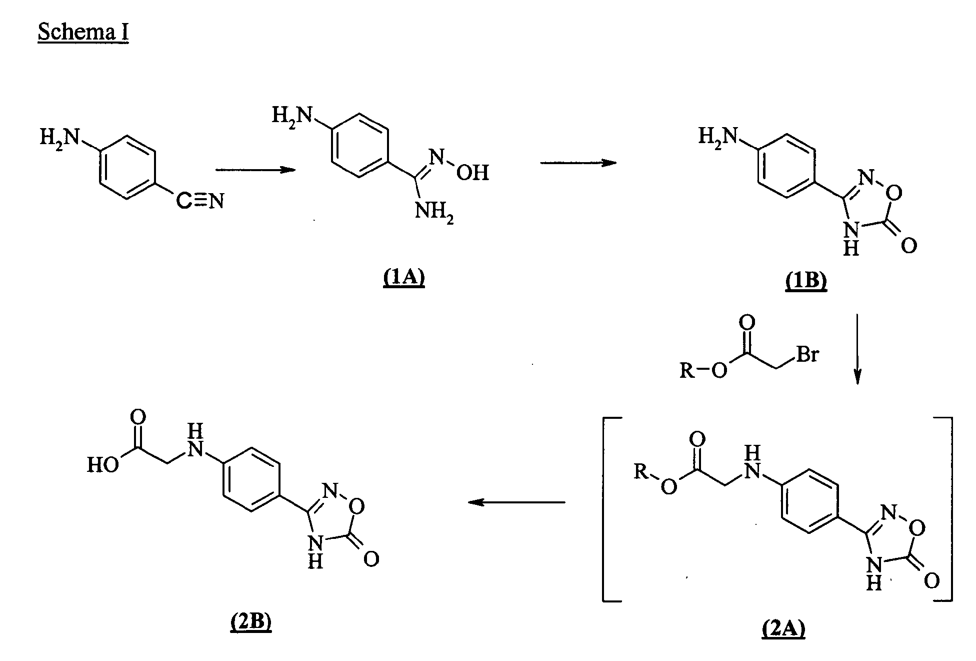

Die Herstellung eines 4-(Benzimidazol-2-ylmethylamino)-benzamidins wird beispielhaft im folgenden Reaktionsschema dargestellt:

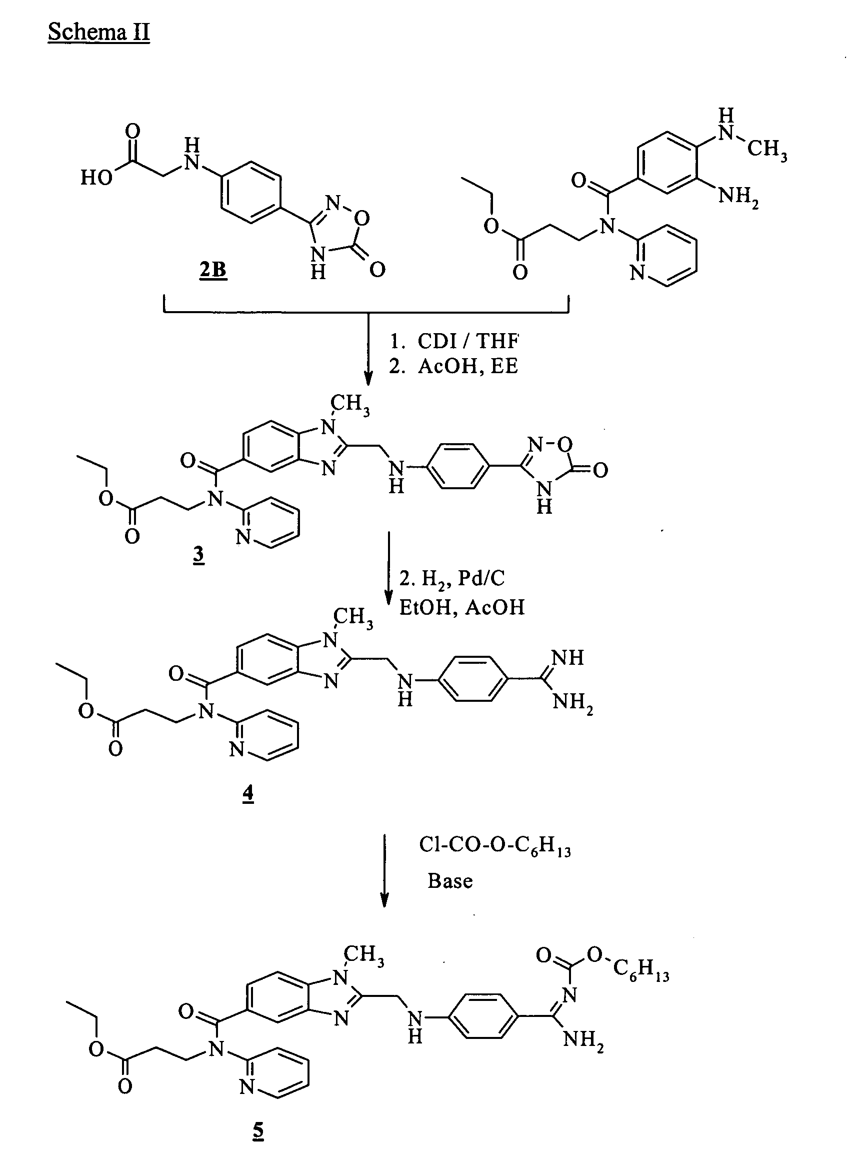

Die Aufarbeitung der einzelnen Reaktionen kann in üblicher Weise erfolgen, zum Beispiel, indem man die Reaktionshilfsmittel abtrennt, das Lösungsmittel entfernt und aus dem Rückstand reines Endprodukt durch Kristallisation, Destillation, Extraktion oder Chromatographie isoliert.

Das erfindungsgemäße Verfahren soll nun durch die nachfolgenden Beispiele erläutert werden. Dem Fachmann ist bewusst, dass die Beispiele nur zur Veranschaulichung dienen und als nicht limitierend anzusehen sind.

### Beispiele

Vor- und nachstehend werden folgende Abkürzungen verwendet:
- AcOH: Essigsäure
- AMBPA: 3-Amino-4-methylaminobenzoesäure-*N*-(2-pyridyl)-*N*-(2- ethoxycarbonylethyl)-amid
- CDI: 1,1'-Carbonyldiimidazol
- EE: Essigsäureethylester
- EtOH: Ethanol
- HCl: Salzsäure
- MtBE: Methyl-*tert*-Butylether
- NaOH: Natriumhydroxid
- PTSA: p-Toluolsulfonsäure
- RT: Raumtemperatur
- THF: Tetrahydrofuran

### Beispiel 1

### Herstellung von 2-[4-(1,2,4-Oxadiazol-5-on-3-yl)-anilin (1):

### (1A)

Im Reaktionsgefäß werden 118,6 g (1 mol) 4-Aminobenzonitril und 68,9 g (0,65 mol) Natriumcarbonat in 500 ml Ethanol und 100 ml Wasser vorgelegt und auf 60°C erwärmt. 76,4 g (1,1 mol) Hydroxylamin Hydrochlorid, gelöst in 100 ml Wasser, werden langsam zu dieser Suspension zugetropft.

Die Mischung wird anschließend über Nacht bei 60°C gerührt. Beim Abkühlen auf 0-5°C fällt die Substanz aus, wird abfiltriert und mehrmals mit insgesamt 150 ml kaltem Wasser und 100 ml kaltem Ethanol nach gewaschen. Abschließend wird mit 50 ml MtBE gewaschen und man erhält 178,4 g feuchtes Produkt. Dieses wird bei 35°C im Vakuum getrocknet.
Ausbeute: 135,4 g hellbeige Substanz (89,5%d.Th), Schmelzpunkt: ab 169,5°C (Zers.); Reinheit: > 98% HPLC-Peakfläche

### (1B)

Zu einer Suspension von 60,5 g **(1A)** (0,4 mol) in 400 ml Ethanol werden portionsweise bei 70-75°C 25,02 g (0,46 mol) Natriummethylat zugegeben und mit 20 ml Ethanol nachgespült.

Nach 15 min Rühren werden 47,25 g (0,4 mol) Diethylcarbonat zugetropft. Nach 3 Stunden Reaktionszeit wird auf 40°C abgekühlt und der Ethanol bei 120 mbar und 40°C abdestilliert. Man erhält einen dunklen Rückstand. Dieser wird bei 40-45°C in 350 ml Wasser gelöst und nach Aufheizen auf 70°C zuerst durch langsame Zugabe von halb konz. Natronlauge auf pH 11; dann durch Ansäuern mit konz. Salzsäure auf pH 5,5 eingestellt und langsam abgekühlt. Die Lösung geht in eine Suspension über, die filtriert und mehrmals mit insgesamt 150 ml kaltem Wasser und 50 ml Ethanol nach gewaschen wird.

Man erhält 88,7 g feuchte Substanz, die bei 35°C im Vakuum getrocknet wird.

Ausbeute: 62 g dunkle Substanz (87,5% d.Th.); Schmelzpunkt: ab 178°C (Zers.); Reinheit: > 98% HPLC-Peakfläche

### Beispiel 2

### Herstellung von 2-[4-(1,2,4-Oxadiazol-5-on-3-yl)-phenylamino]-essigsäure (2):

### (2A)

Bei Raumtemperatur werden 83,5 g (0,5 mol) Bromessigsäureethylester zu einer Suspension von 70,86 g (0,4 mol) **(1B)** und 26,5 g (0,25 mol) Natriumcarbonat in 600 ml Wasser/Isopropanol zudosiert und über Nacht gerührt. Das Reaktionsgemisch wird rötlichbraun bis orange.

Die auf 0°C abgekühlte Suspension wird abgesaugt, in mehreren Portionen mit 300 ml Wasser und 150 ml Ethanol nach gewaschen (106 g feuchte hellbraune Substanz) und bei 35°C im Vakuum getrocknet.

Ausbeute: 92,44 g bräunliche Substanz (87,7% d.Th.) Schmelzpunkt: ab 186,1°C (Zers.) Reinheit: > 98% HPLC-Peakfläche

### (2B)

Der so erhaltene Ester **(2A)** (86,9 g; 0,33 mol) wird in 400 ml Wasser suspendiert und bei RT werden langsam 120 g 45%ige NaOH zugetropft. Die Suspension geht in Lösung und wird rötlich (pH 12,5). Es wird auf ~60°C erwärmt und 1 h verseift. Die erhaltene Lösung wird portionsweise mit 37%iger HCl versetzt, bis pH 5 erreicht ist. Es wird auf 0°C abgekühlt. Der Feststoff wird abgesaugt und in mehreren Portionen mit insgesamt 400 ml kaltem Wasser sowie je 40 ml kaltem Ethanol und MtBE gewaschen. Man erhält 81,4 g feuchte dunkle Substanz. Es wird bei 35°C im Vakuum getrocknet.

Ausbeute: 76,7 g Substanz (98% d.Th.) Schmelzpunkt: ab 193°C (Zers.) Reinheit: > 99% HPLC-Peakfläche

### Beispiel 3

### Herstellung von 1-Methyl-2-[N-[4-(1,2,4-Oxadiazol-5-on-3-yl)phenyl]-amino-methyl]-benzimidazol-5-yl-carbonsäure-N-(2-pyridyl)-N-(2-ethoxycarbonylethyl)-amid (3):

11,35 g (70 mmol) 1,1'-Carbonyldiimidazol werden in 100 ml THF suspendiert und auf 50°C erwärmt. 14,23 g (60,5 mmol) **(2B)** werden portionsweise zugegeben. 17,1 g (50 mmol) AMPBA werden in 37 ml THF unter Erwärmen auf 50°C gelöst.
Nach ca. 90 min wird die Suspension zur Lösung von AMPBA zudosiert und mit 20 ml THF nachgespült.
Die Reaktionsmischung wird ca. 18 h gerührt und anschließend nach Zugabe von 100 ml Essigsäure auf Rückfluss erhitzt, sodass das THF abdestilliert. Nach ca. 1 h wird mit 400 ml Wasser versetzt und gerührt.

Die Lösung wird abgekühlt, die ausgefallene rosafarbene Festsubstanz abfiltriert und mit 20 ml Wasser nach gewaschen in 2 Portionen und bei max 50°C im Vakuum getrocknet. Die isolierte Substanz stellt das Diacetat von (3) dar.

Ausbeute: 24,8 g Substanz (75% d.Th.); Schmelzpunkt: ab 167°C unter Zers. (DSC); Reinheit: > 95% HPLC-Peakfläche

### Beispiel 4

### Herstellung von 1-Methyl-2-[N-[4-amidinophenyl]-amino-methyl]-benzimidazol-5-yl-carbonsäure-N-(2-pyridyl)-N-(2-ethoxycarbonylethyl)-amid (4)

37,3 g (56,4 mmol) **(3)** werden in 900 ml Ethanol gelöst und nach Zugabe von 10 ml Essigsäure und bei 2 bar Wasserstoff mit 4 g wasserfeuchtem 10% Pd/C bei RT hydriert. Man filtriert vom Katalysator und gibt 17 g (89,4 mmol) PTSA, gelöst in 180 ml Ethanol zu. Das Tosylat von **(4)** fällt aus, wird abfiltriert und in mehreren Portionen mit 150 ml Ethanol nachgewaschen.

Man erhält feuchte Substanz, die bei 35°C im Vakuum getrocknet wird.

Ausbeute: 34,5 g hellbeige Substanz (91,3% d.Th.); Schmelzpunkt: 187°C (DSC); Reinheit: > 98% HPLC-Peakfläche.

### Beispiel 5

### Herstellung von 1-Methyl-2-[N-[4-(N-n-hexyloxycarbonylamidino)phenyl]-aminomethyl]-benzimidazol-5-yl-carbonsäure-N-(2-pyridyl)-N-(2-ethoxycarbonylethyl)-amid (5)

Die nach Beispiel 4 erhaltene Verbindung wird in an sich bekannter Weise mit Hexylchloroformiat in Gegenwart einer Base umgesetzt.

## Patentansprüche

1. Verfahren zur Herstellung eines gegebenenfalls substituierten 4-Benzimidazol-2-ylmethylamino)-benzamidins, **dadurch gekennzeichnet, dass** man
(a) ein gegebenenfalls entsprechend substituiertes Diaminobenzol mit 2-[4-(1,2,4-Oxadiazol-5-on-3-yl)-phenylamino]-essigsäure kondensiert,
(b) das so erhaltene Produkt hydriert, und
(c) gegebenenfalls die Amidinogruppe carbonyliert.

2. Verfahren nach Anspruch 1 zur Herstellung eines gegebenenfalls substituierten 4-Benzimidazol-2-ylmethylamino)-benzamidins der Formel (I) 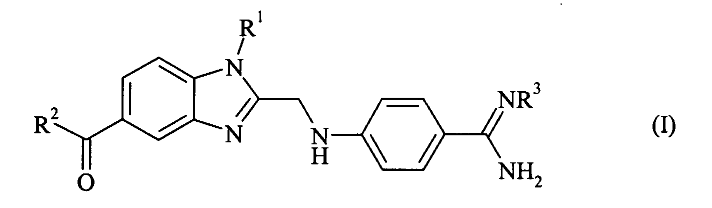 worin
R¹ für C₁₋₆-Alkyl- oder C₃₋₇-Cycloalkylgruppe steht,
R²
(i) für eine C₁₋₆-Alkylgruppe, eine gegebenenfalls durch eine C₁₋₃-Alkylgruppe substituierte C₃₋₇-Cycloalkylgruppe, wobei die C₁₋₃-Alkylgruppe zusätzlich durch eine Carboxylgruppe oder durch eine *in-vivo* in eine Carboxygruppe überführbare Gruppe substituiert sein kann,
steht, oder
(ii) für eine R²¹NR²²-Gruppe steht, in der
R²¹ eine C₁₋₆Alkylgruppe, die durch eine Carboxy-, C₁₋₆ Alkoxycarbonyl-, Benzyloxycarbonyl-, C₁₋₃-Alkylsulfonylaminocarbonyl-, Phenylsulfonylaminocarbonyl-, Trifluorsulfonylamino-, Trifluorsulfonylaminocarbonyl- oder 1H-Tetrazolylgruppe substituiert sein kann, eine durch eine Hydroxy-, Phenyl-C₁₋₃-alkoxy-, Carboxy-C₁₋₃-alkylamino-, C₁₋₃-Alkoxycarbonyl-C₁₋₃-alkylamino-, N-(C₁₋₃-Alkyl)-carboxy-C₁₋₃-alkylamino- oder N-(C₁₋₃-Alkyl)-C₁₋₃-alkoxycarbonyl-C₁₋₃-alkylaminogruppe substituierte C₂₋₄-Alkylgruppe, wobei in den vorstehend erwähnten Gruppen das zum benachbarten Stickstoffatom stehende α-Kohlenstoffatom nicht substituiert sein kann, oder eine gegebenenfalls durch eine C₁₋₃-Alkylgruppe substituierte Piperidinylgruppe bedeutet, und
R²² ein Wasserstoffatom, eine C₁₋₆-Alkylgruppe, eine gegebenenfalls durch eine C₁₋₃-Alkylgruppe substituierte C₃₋₇-Cycloalkylgruppe, eine C3₋₆-Alkenyl- oder C₃₋₆-Alkinylgruppe, wobei der ungesättigte Teil nicht direkt mit dem Stickstoffatom der R²¹NR²²-Gruppe verknüpft sein kann, eine gegebenenfalls durch ein Fluor-, Chlor- oder Bromatom, durch eine C₁₋₃-Alkyl- oder C₁₋₃-Alkoxygruppe substituierte Phenylgruppe, eine gegebenenfalls durch eine C₁₋₃-Alkylgruppe substituierte Benzyl-, Oxazolyl-, Isoxazolyl-, Thiazolyl-, Isothiazolyl-, Pyrazolyl-, Pyridinyl-, Pyrimidinyl-, Pyrazinyl-, Pyridazinyl-, Pyrrolyl-, Thienyl- oder oder Imidazolylgruppe bedeutet,oder
R²¹ und R²² zusammen mit dem dazwischenliegenden Stickstoffatom eine gegebenenfalls durch eine Carboxy- oder C₁₋₄-Alkoxycarbonylgruppe substituierte 5- bis 7-gliedrige Cycloalkyleniminogruppe, an welche zusätzlich ein Phenylring ankondensiert sein kann, darstellen,
und
R³ für ein Wasserstoffatom, eine C₁₋₉-Alkoxycarbonyl-, Cyclohexyloxycarbonyl-, Phenyl-C₁₋₃-alkoxycarbonyl-, Benzoyl-, p-C₁₋₃-Alkyl-benzoyl- oder Pyridinoylgruppe steht, wobei der Ethoxyteil in 2-Stellung der vorstehend erwähnten C₁₋₉-Alkoxycarbonylgruppe zusätzlich durch eine C₁₋₃-Alkylsulfonyl- oder 2-(C₁₋₃-Alkoxy)-ethylgruppe substituiert sein kann, darstellt,
wobei man im Schritt (a) ein Phenyldiamin der Formel (II) 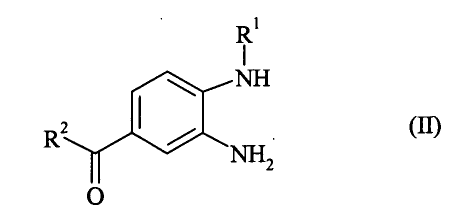 worin R¹ und R² die für Formel (I) angegebene Bedeutung aufweisen,
mit 2-[4-(1,2,4-Oxadiazol-5-on-3-yl)-phenylamino]-essigsäure umsetzt, das erhaltene Produkt der Formel (III) 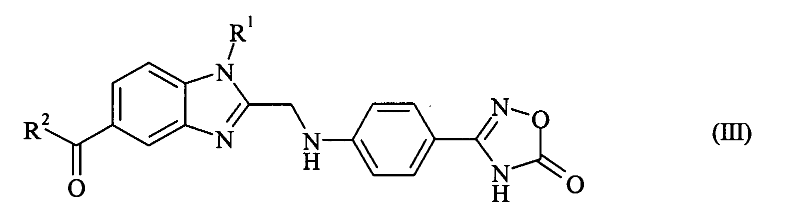 worin R¹ und R² die für Formel (I) angegebene Bedeutung aufweisen,
in Schritt (b)hydriert, und
(c) gegebenenfalls die so erhaltene Verbindung der Formel (I), worin R³ Wasserstoff bedeutet, mit einer Verbindung der Formel (IV)
R³-X (IV)
worin R³ die für Formel (I) angegebene Bedeutung aufweist, und
X für eine geeignete Abgangsgruppe steht,
umsetzt.

3. Verfahren nach Anspruch 1 oder 2, zur Herstellung einer Verbindung der Formel (I), worin
R¹ für eine C₁₋₃-Alkylgruppe steht,
R² für eine R²¹NR²²-Gruppe steht, in der
R²¹ eine C₁₋₃Alkylgruppe, die durch eine Carboxy-, C₁₋₃ Alkoxycarbonyl-, substituiert sein kann,
bedeutet, und
R²² ein Wasserstoffatom, eine C₁₋₃-Alkylgruppe, eine gegebenenfalls durch eine C₁₋₃-Alkylgruppe substituierte Pyridinylgruppe bedeutet,
und
R³ für ein Wasserstoffatom, eine C₁₋₈-Alkoxycarbonylgruppe steht.

4. Verfahren nach Anspruch 3, zur Herstellung der Verbindung der Formel (I), worin
R¹ für eine Methylgruppe steht,
R² für eine R²¹NR²²-Gruppe steht, in der
R²¹ eine Ethylgruppe, die durch eine Ethoxycarbonylgruppe substituiert ist, bedeutet, und
R²² Pyridin-2-ylgruppe bedeutet,
und
R³ für eine Hexyloxycarbonylgruppe steht.

5. Verfahren nach einem der vorangehenden Ansprüche, **dadurch gekennzeichnet, dass** die Kondensation des Schrittes (a) in Gegenwart eines inerten Verdünnungsmittels und eines wasserbindenden Mittels durchführt.

6. Verfahren nach einem der vorangehenden Ansprüche, **dadurch gekennzeichnet, dass** man die Hydrierung des Schrittes (b) in Gegenwart eines inerten Verdünnungsmittels und eines Hydrierkatalysators durchführt.

7. Verfahren nach einem der vorangehenden Ansprüche, **dadurch gekennzeichnet, dass** man zur Herstellung von 2-[4-(1,2,4-Oxadiazol-5-on-3-yl)-phenylamino]-essigsäure 2-[4-(1,2,4-Oxadiazol-5-on-3-yl)-anilin mit einem 2-Halogenessigsäureester in Gegenwart einer schwachen Base umsetzt, und den erhaltenen 2-[4-(1,2,4-Oxadiazol-5-on-3-yl)-phenylamino]-essigsäureester verseift.

8. Verfahren nach Anspruch 8, **dadurch gekennzeichnet, dass** man zur Herstellung von 2-[4-(1,2,4-Oxadiazol-5-on-3-yl)-anilin 4-Aminophenyl-amidoxim mit einem Dialkylcarbonat in Gegenwart einer Base umsetzt.

9. Verbindung der Formel (III) 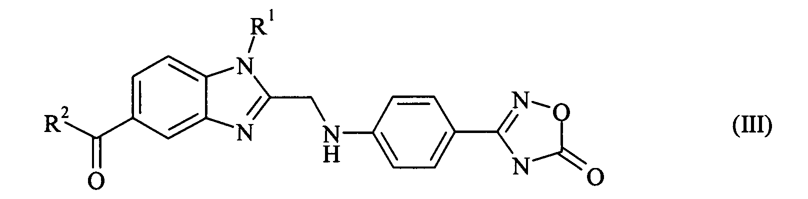 worin R¹ und R² die in einem der Ansprüche 2 bis 4 angegebene Bedeutung aufweisen.

10. 2-[4-(1,2,4-Oxadiazol-5-on-3-yl)-phenylamino]-essigsäure.

11. 2-[4-(1,2,4-Oxadiazol-5-on-3-yl)-anilin.
